Europäisches Patentamt

European Patent Office

Office européen des brevets

(11) Publication number: **0 166 585**
**A2**

# (12) EUROPEAN PATENT APPLICATION

(21) Application number: 85304445.1

(22) Date of filing: 21.06.85

(51) Int. Cl.⁴: **C 12 N 15/00**, C 12 P 21/02, C 07 K 13/00, A 61 K 37/02, A 61 K 37/24, C 12 N 1/20 // (C12N1/20, C12R1:19)

(30) Priority: 22.06.84 JP 127273/84
22.06.84 JP 127274/84

(43) Date of publication of application: 02.01.86
Bulletin 86/1

(84) Designated Contracting States: AT BE CH DE FR GB IT LI LU NL SE

(71) Applicant: Suntory Limited, 1-40 Dojimahama 2-chome Kita-ku, Osaka-shi Osaka-fu 530 (JP)
Applicant: Matsuo, Hisayuki, 6653 Ooaza-Kihara Kiyotake-cho, Miyazaki-gun Miyazaki-ken (JP)

(72) Inventor: Matsuo, Hisayuki, 6653 Ooaza Kihara Kiyotake-cho, Miyazaki-gun Miyazaki-ken (JP)
Inventor: Kangawa, Kenji, 1520-24 Kanoukou Kiyotake-cho, Miyazaki-gun Miyazaki-ken (JP)
Inventor: Hayashi, Yujiro, 31-2, Ikeda 1-chome, Neyagawa-shi Osaka (JP)
Inventor: Oikawa, Shinzo, 1-8-703 Ooharano-higashisakaidani 1-chome, Nishikyo-ku, Kyoto-shi, Kyoto (JP)
Inventor: Oshima, Takehiro, 17-10-383 Besshohonmachi, Takatsuki-shi Osaka (JP)
Inventor: Tanaka, Shoji, 8-46 Minamikaneden-cho 1-chome, Suita-shi Osaka (JP)
Inventor: Nakazato, Hiroshi, Misawa-hai-taun E-505, 13 Misawa-cho, Ibaraki-shi Osaka (JP)
Inventor: Tawaragi, Yasunori, Rabiannu-REPO 203 1-10 Shimohozumi 2-chome, Ibaraki-shi Osaka (JP)

(74) Representative: Ford, Michael Frederick et al, MEWBURN ELLIS & CO. 2/3 Cursitor Street, London EC4A 1BQ (GB)

(54) Novel peptide and gene coding for same.

(57) A DNA fragment including a base sequence coding for a peptide occurring in rat atrium cordis and having diuretic action or a precursor of the peptide, plasmids containing the DNA fragment, microorganisms transformed with the plasmid, and a process for production of the peptide using the transformant.

A new peptide consisting of 126 amino acids occuring in rat atrium cordis and a precursor thereof. The peptide has notable diuretic action and hypotensive or antihypertensive actions.

EP 0 166 585 A2

- 1 -

## NOVEL PEPTIDE AND GENE CODING FOR SAME

The present invention relates to a novel peptide, a gene coding for the peptide, a process for production of the peptide using the gene inserted to an expression vector, and use of the peptide.

Normal regulation of the blood pressure in a human body is important for the maintenance of personal health. Various physical and humoral factors contribute to this regulation of the blood vessels, etc. The humoral factors include, for example, the reninangiotensin-aldsterone system, catecholamines, prostaglandins, kinin-kallikrein system, and natriuretic hormones including ouabain-like substances. Herein the term "natriuretic" will denote selective excretion of sodium cation relating to potassium cation.

Granules morphologycally similar to granules present in peptide hormone-producing cells are found in human atrium (J.D. Jamieson and G.E. Palade, J. Cell Biol., 23, 151,. 1964). An extract of rat atrium and granules contained therein are known to show natriuretic action in rats (A.J. DeBold et. al., Life Science, 28, 89, 1981; R. Keeller, Can. J. Physiol. Pharmacol., 60, 1078, 1982). Recently Mark G., Currie S. et. al. suggested peptide-like substances with a molecular weight of 20,000 to 30,000, or not more than 10,000, present in the atrium of humans, rabbits, swine, and rats, and having natriuretic action (Science, 221, 71-73, 1983).

Moreover, a peptide consisting of 28 amino acid derived from rat atrium cordis was identified (Biochem; Biophys. Res. Commun., vol 117, No. 3, P 859-865, 1983; Japanese Unexamined Patent Publication No. 59-157026 (designated in the present application as

α-rANP). The present inventors found a new peptide consisting of 28 amino acids from human atrium cordis, referred to as "α-human atrial natriuretic polypeptide" and abbreviated as "α-hANP" (Biochem. Biophys. Res. Commun. Vol 118, No. 1, P 131-139, 1984, U.S.P. Serial No. 685151 of the present inventors. The α-hANP has the following amino acid sequence:

```
      1                                    10
    H-Ser-Leu-Arg-Arg-Ser-Ser-Cys-Phe-Gly-Gly-Arg-Met-
                                  20
    Asp-Arg-Ile-Gly-Ala-Glu-Ser-Gly-Leu-Gly-Cys-Asn-
                28
    Ser-Phe-Arg-Tyr-OH
```

wherein Cys at the 7 position and Cys at the 23 position are bonded through a disulfide bond.

Various kinds of physiologically active peptides are known to be derived in vivo from their precursors by the action of an endopeptidase or exopeptidase, rather than produced directly. For example, β-endorphin and γ-liportropin are derived from their precursor β-lipotropin, and β-melanocyte-stimulating hormone is derived from its precursor γ-lipotropin (Takahashi H. et. al., FEBS Lett. 135, 97-102, 1981).

On the basis of the above-mentioned findings, the present inventors expected that the above-mentioned α-rANP would be derived from its precursor and actually found a new peptide derived from rat atrium cordis consisting of 126 amino acids including 28 amino acids of the α-rANP. Moreover, the present inventors successfully obtained a gene coding for the new peptide and constructed plasmid containing the gene which expresses in a microorganism.

Therefore, the present invention provides a new peptide consisting of 126 amino acids and having diuretic action and hypotensive or antihypertensive action. The peptide is hereinafter referred to as "γ-rat atrial natriuretic polypeptide" and abbreviated as γ-rANP".

There is also provided a DNA fragment comprising a base sequence coding for a peptide occurring in rat atrium cordis and having diuretic action or a precursor of the peptide.

There is also provided plasmids containing a promotor region, SD sequence, and the above-mentioned DNA fragment and capable of producing a peptide coded by the DNA fragment in a microoganism.

There is also provided a microorganism transformed with the plasmid.

The present invention further provides a process for the production of the γ-rANP or precursor peptide thereof by culturing the transformed microorganism in a culture medium and recovering the γ-rANP or precursor from the cultured cells or medium, or converting either such peptide to or from an acid addition salt.

The present invention further extends to provide a pharmaceutical composition containing the γ-rANP as a diuretic or hypotensor and extends to medical use of γ-rANP.

The invention will be further explained and embodiments and examples set forth, in the description below, referring to the drawings in which:

Figure 1 shows an amino acid sequence of γ-rANP consisting of 126 amino acids and optional arginine residues X.

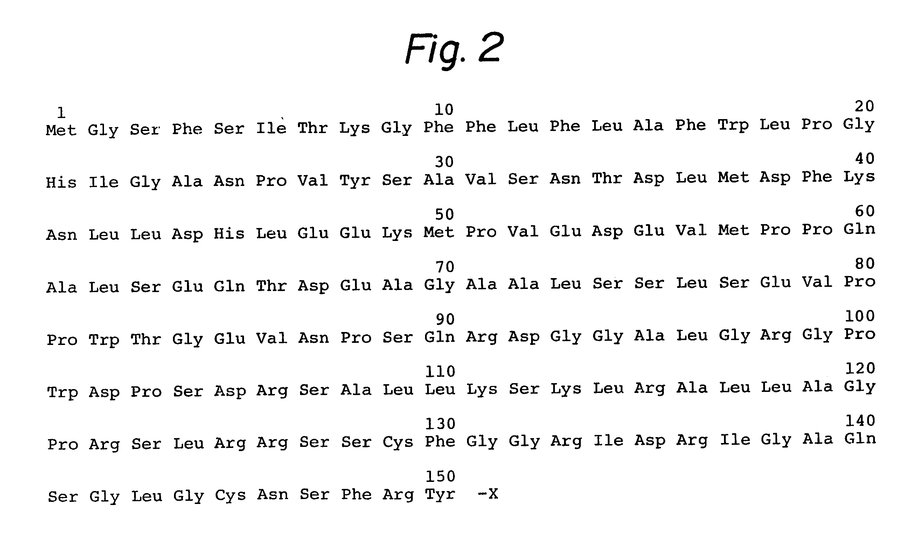

Fig. 2 shows an amino acid sequence of a precursor of the γ-rANP, which sequence consists of 151 amino acids and optional arginin residues X wherein a sequence of from 25 to 150 corresponds to the sequence of the γ-rANP set forth in Fig. 1;

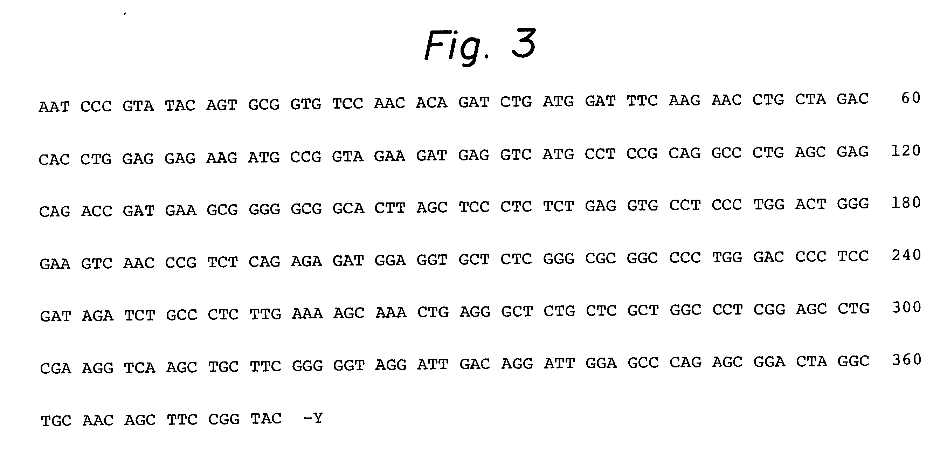

Fig. 3 shows a base sequence coding for the amino acid sequence set forth in Fig. 1;

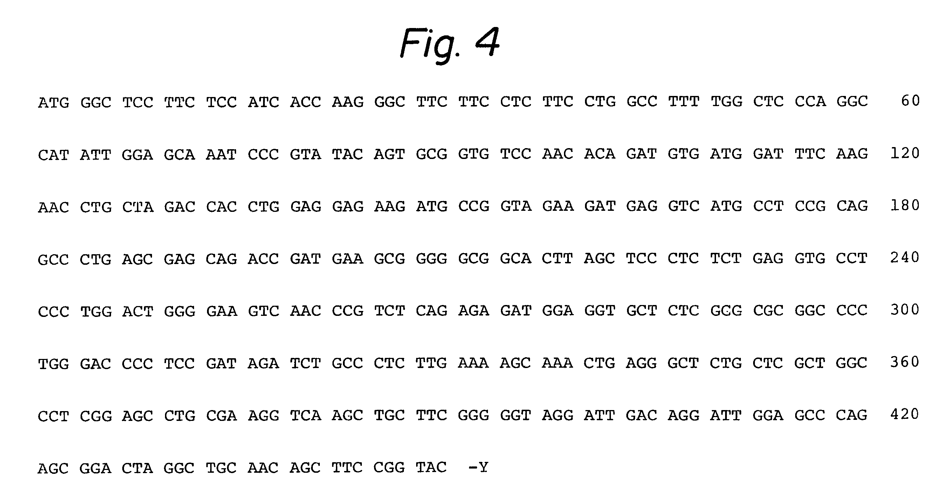

Fig. 4 shows a base sequence coding for the amino acid sequence set forth in Fig. 2;

Fig. 5 shows base sequences of probes (I) and (II) used for dideoxy claim termination sequencing method;

Fig. 6 shows a strategy used for sequencing a cDNA fragment cloned into a plasmid prANP10;

Fig. 7 shows a base sequence of a cDNA fragment cloned into the plasmid prANP10 including a 5' non-coding region, a coding region corresponding to the base sequence set forth in Fig. 4, and a 3' non-coding region, and an amino acid sequence corresponding to the coding region;

Fig. 8 shows a construction process of plasmid pS320 from plasmid pS220 and a DNA fragment containing a base sequence coding for amino acid sequence of γ-rANP-arg and a construction process of plasmid pS324-3 from plasmid pS224-3 and the same DNA fragment;

Fig. 9 shows a construction process of plasmid pS325 from plasmid pS220 and plasmid pS320 and a construction process of plasmid pS329-3 from plasmid pS224-3 and plasmid pS324-3;

Fig. 10 shows base sequences near stop codons in plasmids pS220, pS320, and pS325;

Fig. 11 shows results of electrophoresis showing the expression of a γ-rANP-arg gene;

Fig. 12 shows graphs of the diuretic action of γ-rANP;

Fig. 13 is a chart of the hypotensive action of the γ-rANP;

Fig. 14 is a chromatogram of an elution profile using Sephadex G-75 during the purification of the γ-rANP; and

Fig. 15 represents an elution profile of high performance liquid chromatography (HPLC) during the purification of the γ-rANP.

1.    Identification of γ-rANP Gene

RNA were extracted from Wister Kyoto rat artrium cordis by a method disclosed in Chirgwin (Chirgwin, J. M. et. al., Biochemistry 18, 5294-5299, 1979), and enriched for poly (A)$^+$ RNA (mRNA) with an oligo (dT) cellulose column. The poly (A)$^+$ RNA were used to prepare a cDNA library according to the Okayam-Berg method (Mol. Cell. Biol. $\underline{2}$, 161-170, 1982). For screening of the cDNA library thus obtained, a probe was prepared by nick translation of cDNA of γ-hANP with $^{32}$P (Rigby, P. W. et. al., J. Mol. Biol., $\underline{113}$, 237, 1977). The cDNA of γ-hANP was disclosed in Japanese Patent Application No. 59-116605. As an amino acid sequence of the γ-hANP was similar to that of γ-rANP as described whereinafter, it was reasonably expected that the probe derived from the cDNA of γ-hANP would be useful as a probe for screening the library to select cDNA of γ-rANP gene.

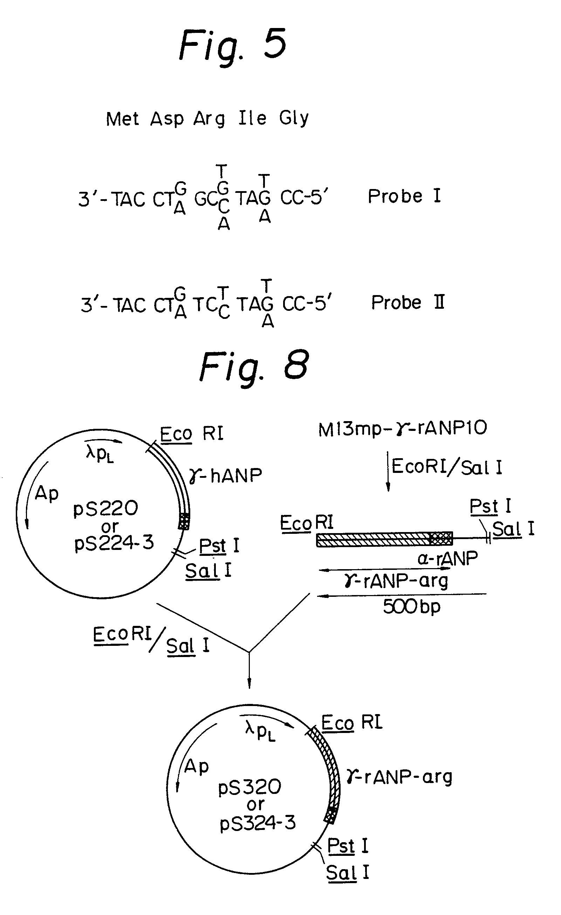

From a cDNA library containing about 15,000 clones, about 50 clones which hybridized with the probe were selected. Among the 50 clones, 12 clones were used to obtain plasmids. Each plasmid was cleaved with restriction endonucleases Pvu II and Pst I to obtain a DNA fragment corresponding to the cDNA inserted by the Okayama-Berg method described above. Each cDNA fragment thus obtained was tested by gel electrophoresis, and three DNA fragments were found to be 800 base pairs (bp) to 950 bp in length. Three plasmids from which the above-mentioned three DNA fragments were derived were designated prANP 10, prANP 11, and prANP 12. The above-mentioned three cDNA fragments were sequenced using a mixture of chemically synthesized 14-mer oliganucleodides (Fig. 5) corresponding to an amino acid sequence of from 12 (methionine) to 16 (glycine) of α-hANP as a primer, according to the dideoxy chain termination method (Sanger, F. et. al., Proc. Natl. Acad. Sci. USA, 5463-5467, 1977). As a result, the three DNA fragments were found to contain a base sequence corresponding to an amino acid sequence of the first to 11th amino acids of α-rANP. The plasmid prANP 10, which is confirmed to contain the longest cDNA fragment, was chosen for further investigation.

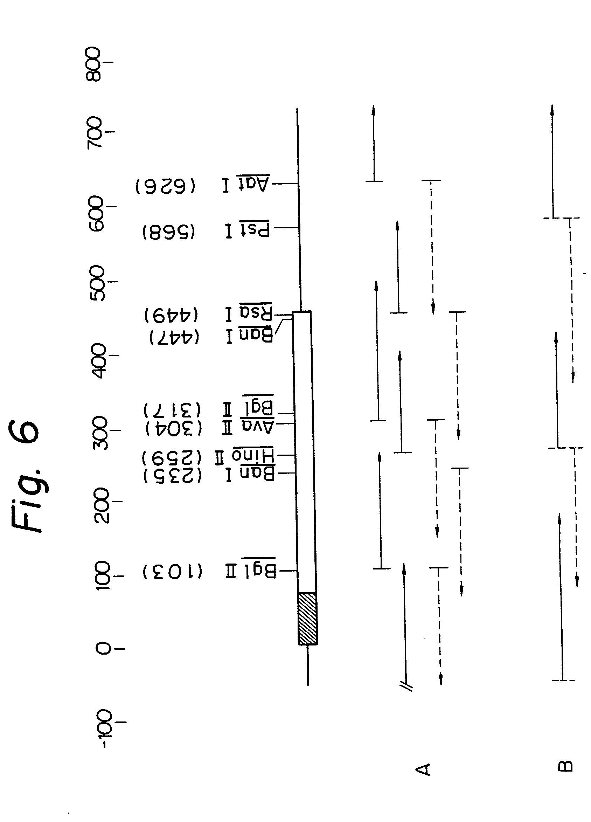

The entire cDNA sequence in the plasmid prANP 10 was determined according to the Maxam and Gilbert method (Meth. Enzym., 65, 499-560, 1980) and the method disclosed by Sanger et. al. (Proc. Natl. Acad. Sci. USA, 74, 5463-5467, 1977). To carry out the sequencing procedures, a strategy shown in Fig. 6 was used.

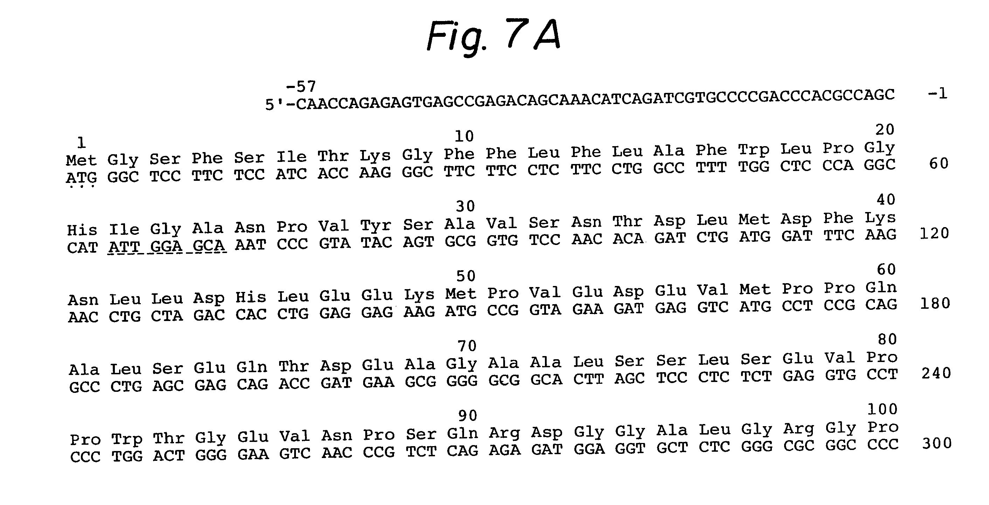

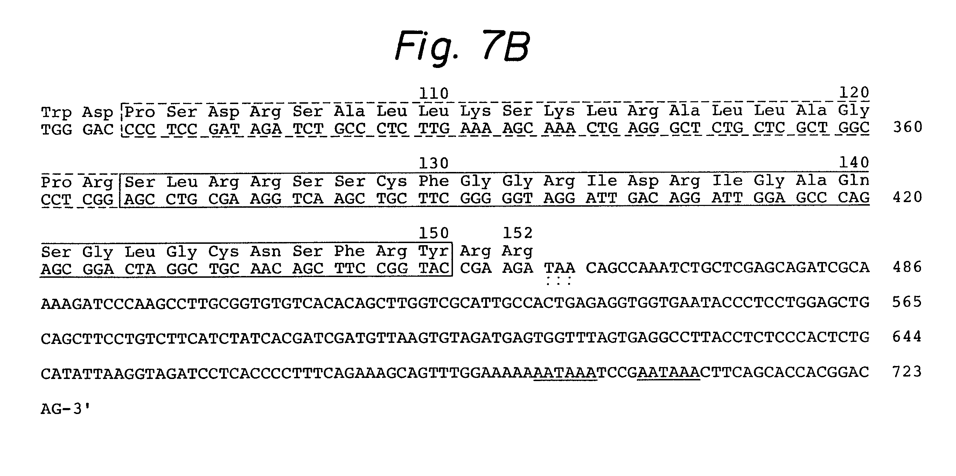

A base sequence thus determined of the cDNA fragment in the plasmid prANP 10 is set forth in Fig. 7. As seen from Fig. 7, the cDNA fragment consists of a 5' non-translation region consisting of 57 bp; an open reading frame starting from a starting codon ATG shown by three dots and ending before a translation stop codon TAA shown by six dots; and a 3' non-translation region consisting of 269 bp including the stop codon. Figure 7 also contains the amino acid sequence expected from the base sequence of the open reading frame. The open reading frame codes for an amino acid sequence consist of 152 amino acid. The expected amino acid sequence includes an amino acid sequence of α-rANP already established, which sequence is shown by the solid line box in Fig. 7. .The expected amino acid sequence also includes an amino acid sequence of β-rANP shown by a dotted line box and the subsequent solid line box of from amino acid position 103 to amino acid position 150 (Japanese Patent Application No. 59-038816).

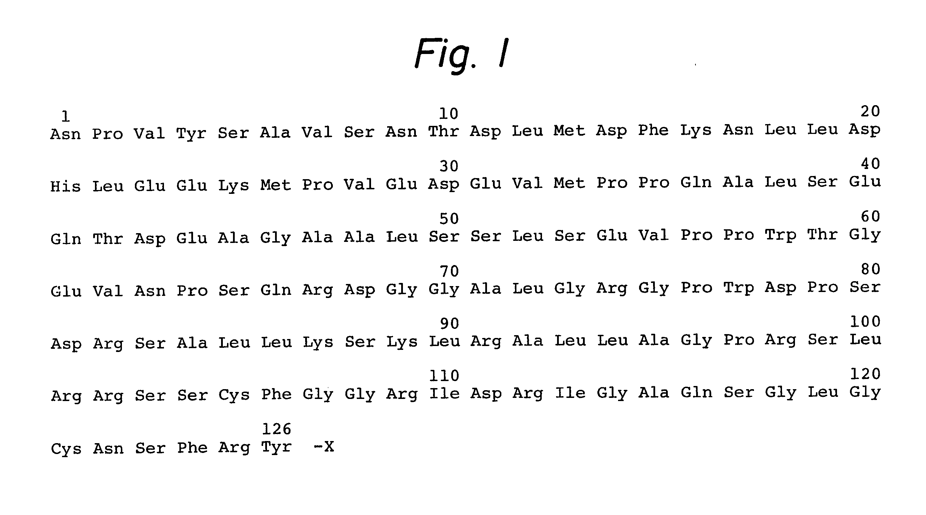

On the other hand, a peptide having a molecular weight of about 13,000 Dalton was isolated from rat atrium cordis. The peptide was sequenced, and determined to have an amino acid sequence set forth in Fig. 1 but not containing -X (described below in detail).

The amino acid sequence of the peptide extracted from rat atrium cordis is identical with an expected amino acid sequence from the 25 position to 150 position in Fig. 7. This means that the cDNA fragment in the plasmid prANP10 contains a base sequence coding for

γ-rANP. The cDNA fragment also contains an additional base sequence coding for an amino acid sequence from Met at position 1 to Ala at position 24 in Fig. 7. The amino acid sequence from 1 to 24 is believed to be a signal peptide which participates in the secretion of a peptide.

The above-mentioned relationship between α-rANP, and γ-rANP supports the expectation that γ-rANP is a precursor of α-rANP. When α-rANP is derived from γ-rANP, a C-end amide bond of the Pro-Arg sequence present at 121 and 122 in Fig. 7, i.e., an amide bond between Agr-Ser at 122 and 123, has to be cleaved. This possibility is strongly suggested from a phenomenon wherein gastrin releasing peptide is derived from its precursor by cleavage of a C-end amide bond of the Pro-Arg sequence present in the precursor (Reave, J.R. et. al., J. Biol. Chem. 258, 5582-5588, 1983; and Minamino, N. et. al., Biochem. Biophs. Res. Commun. 119, 14-20, 1984). From the similarity between γ-rANP and gastrin releasing peptide in the presence of the Pro-Arg sequence, α-rANP is reasonably considered to be formed in vivo from γ-rANP by processing (perhaps by enzymic action).

In the above-mentioned relationship between β-rANP and γ-rANP, β-rANP is believed to derive from γ-rANP by cleavage of C-end of Trp-Asp of amino acid positions 101 and 102 in Fig. 7. While at present the mechanism which cleaves this kind of site is not known, a mechanism is supposed to be present.

The cDNA fragment also contained two codons coding for arginine between a codon TAC for tyrosine at amino acid position 150, which is a C-terminal amino acid of γ-rANP and the translation stop codon TAA. From the presence of two codons for arginine, it is supposed that γ-rANP is produced in vivo from a precursor having two additional arginines at the C-end of γ-rANP by deletion of an Arg-Arg-OH sequence with, for example, a carboxypeptidase B-like enzyme. The supposed precursor having two arginines at the C-end of γ-rANP is designated here as "γ-rANP-arg".

2. Construction of Expression Plasmid

As a DNA fragment cloned into plasmid prANP10 contains a base sequence coding for γ-rANP, and therefore, β-rANP and α-rANP, which have natriuretic or diuretic action and hypotensive or antihypertensive action, the DNA fragments are useful for the industrial production of γ-rANP, β-rANP, α-rANP and other diuretic peptides having an amino acid sequence present in the γ-rANP.

For the construction of an expression plasmid containing a base sequence coding for γ-rANP and useful for expression of the γ-rANP gene from the cDNA fragment set forth in Fig. 7, two kinds of manipulations of the cDNA are necessary, i.e., (a) insertion of a restriction enzyme cleavage site and a translation initiation codon ATG into an upstream site adjacent to the first codon of the γ-rANP gene in the DNA fragment and (b) insertion of a translation stop codon downstream site adjacent to the last codon of the γ-rANP gene. The former was accomplished by _in vitro_ mutation; and the latter was accomplished by two steps, i.e., construction of an expression plasmid for γ-rANP-arg and conversion of the expression plasmid for γ-rANP-arg to a expression plasmid for γ-rANP.

First, a restriction enzyme _EcoRI_ cleavage site and a translation initiation codon ATG were incorporated into an upstream site adjacent to the γ-rANP gene in the DNA fragment by _in vitro_ mutation (Zoller, M. J. & Smith, M., Nucl. Acid. Res. _10_, 6487, 1982). RF-DNA of M13mp8 and DNA of plasmid prANP10 were cleaved with _Pst_ I to obtain cleaved RF-DNA of M13mp8 and a DNA fragment of about 650 bp containing the γ-rANP-arg gene, respectively. Both DNA fragments were ligated with T4 DNA ligase and transformed into _E. coli_ JM103. The transformed _E. coli_ was cultured to obtain a single strand phage containing the 650 bp fragment. The single strand phage DNA was used to form a hetero duplex with a chemically synthesized 33 mer single strand DNA. The synthesized DNA consists of a base sequence GAATTCATG coding for the above-mentioned EcoRI site and the translation initiation codon ATG and corresponding to a sequence ATT GGA GCA in Fig. 7 (shown by a dotted line), a base sequence of 12 bases identical with a sequence upstream of the dot-lined sequence, and a base sequence of 12 bases identical with a sequence downstream of the dot-lined sequence. The single strand DNA partially having the hetero duplex was converted to a double strand DNA according to a conventional method with a DNA polymerase I Klenow fragment (Zoller, J.M. & Smith, M., Nucl. Acid Res. 10, 6487, 1982). The formed double strand DNA was transformed into E. coli JM103. To select plasmid DNA containing a base sequence GAATTCATG in place of the original base sequence ATTGGAGCA, screening was carried out by culturing the transformed E. coli, obtaining DNA, and cleaving the DNA with EcoRI. A base sequence covering the sequence GAA TTC ATG was determined by a dideoxy chain termination method (Sanger, F. et. al., Proc. Natl. Acid. Sci. USA 74, 5463-5467, 1977), and a plasmid containing a desired sequence was selected and designated as M13mp8-γ-rANP10.

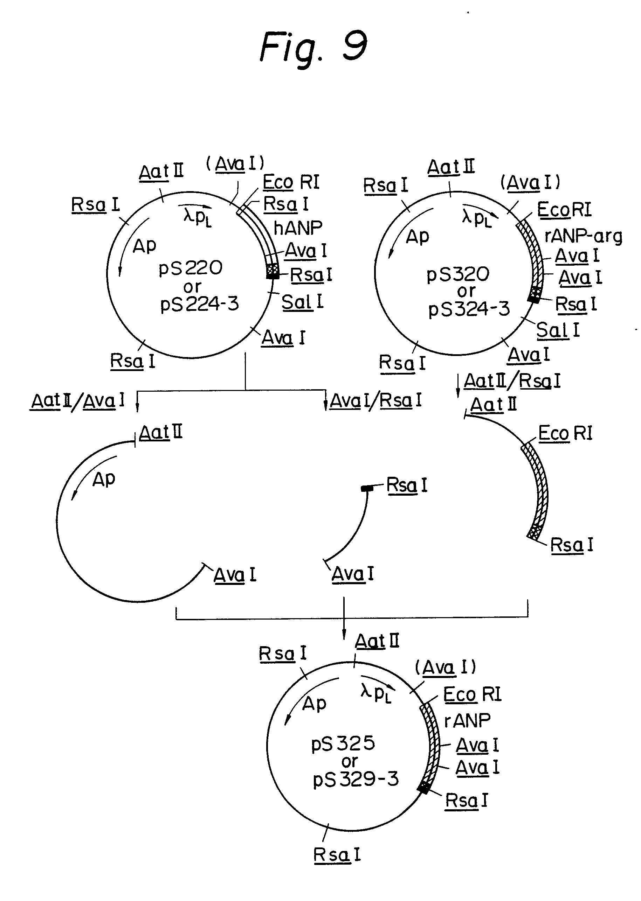

To construct an expression plasmid, the plasmid M13mp8-γ-rANP10 was cleaved with Eco RI and Sal I to obtain a DNA fragment of about 500 bp containing the γ-rANP gene. The Eco RI cleaves the Eco RI cleavage site incorporated by the above-mentioned in vitro mutation; and the Sal I cleaves a Sal I cleavage site originally present in M13mp8. A plasmid pS220 (Fig. 8) was cleaved with Eco RI, and Sal I, and a Eco RI - Sal I DNA fragment of about 500 bp was replaced with the above-mentioned Eco RI - Sal I fragment of about 500 bp to obtain an expression plasmid pS320 for γ-rANP-arg (Fig. 8). According to a similar procedure, an expression plasmid pS324-3 for γ-rANP-arg was constructed from the plasmid M13mp8-γ-rANP-10 and a plasmid pS 224-3 (Fig. 8).

The starting plasmids pS220 and pS224-3, which are expression plasmids for γ-hANP, were constructed by insertion of a DNA fragment containing a γ-hANP gene into plasmid pS20 and pS84-3 respectively as described hereinafter. The plasmid pS20 has a $\lambda P_L$ promotor and SD sequence of a λ phage CII protein gene as a non-translation region followed by an Eco RI cleavage site and can express a foreign gene inserted at the Eco RI site under the control of the $\lambda P_L$ promotor. The plasmid pS20 has been constructed by the inventors and deposited at the Fermentation Research Institute of the Agency of Industrial Science and Technology (FRI) under the Budapest Treaty on the International Recognition of the Deposit of Microorganisms for the Purposes of Patent (Budapest Treaty) as FERM BP-535 on May 30, 1984.

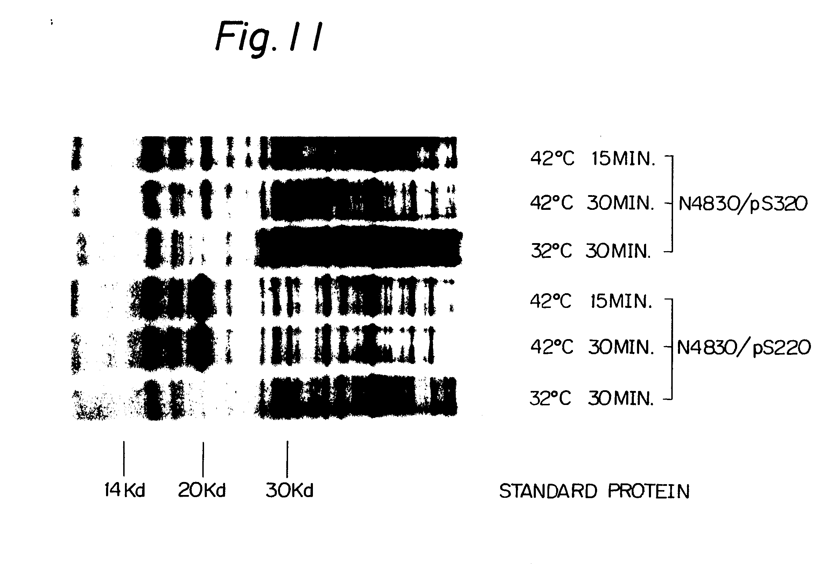

The plasmid pS320 was transformed into E. coli N4830, and the transformed strain was cultured in a minimum medium M9 supplemented with 50 µg/ml ampicillin and 19 natural amino acids except for L-methionine at a temperature of 32°C. When the E. coli was grown to $OD_{660}$ = 0.4, the temperature was shifted from 32°C to 42°C. At 15 minutes and 30 minutes after the shift of temperature, 0.3 ml of culture was obtained from the culture, and to the culture (0.3 ml), 2 µCi of $^{35}$S-methionine with 800 Ci/m mole of specific activity (New England Nuclea) was added. Culturing was continued for 2 minutes. A control culture was prepared with E. coli N4830 not containing plasmid pS320. Proteins were extracted and analyzed with SDS-polyacrylamide gel electrophoresis (SDS-PAGE) to determine the composition of the proteins. The culture of E. coli containing plasmid pS320 provided a band of slightly higher than 20Kd which was not found in the control culture (Fig. 11). This means that the plasmid pS320 expressed a new protein with about 20Kd. Similarly, E. coli N4830 was transformed with the plasmid pS324-3 to obtain a transformant N4830/pS324-3. The transformant was also confirmed to produce a new protein with a molecular weight slightly higher than 20Kd on the SDS-PAGE.

While the molecular weight shown on the SDS-PAGE is higher than a theoretical molecular weight of γ-rANP-arg, the protein was determined to be γ-rANP-arg because γ-rANP prepared from rat atrium cordis migrates to a site corresponding to about 20Kd on the SDS-PAGE and because the SDS-PAGE often shows a molecular weight higher than a theoretical molecular weight for other proteins.

The plasmid pS320 contains a $\lambda P_L$ promotor, a DNA sequence for a part of N' protein of λ phage and an SD sequence of CII protein, a γ-rANP-arg gene, and a translation stop codon, in that order. The plasmid pS324-3 contains a $\lambda P_L$ promotor, an SD sequence of MS2 phage A protein, a γ-rANP-arg gene, and a translation stop codon, in that order.

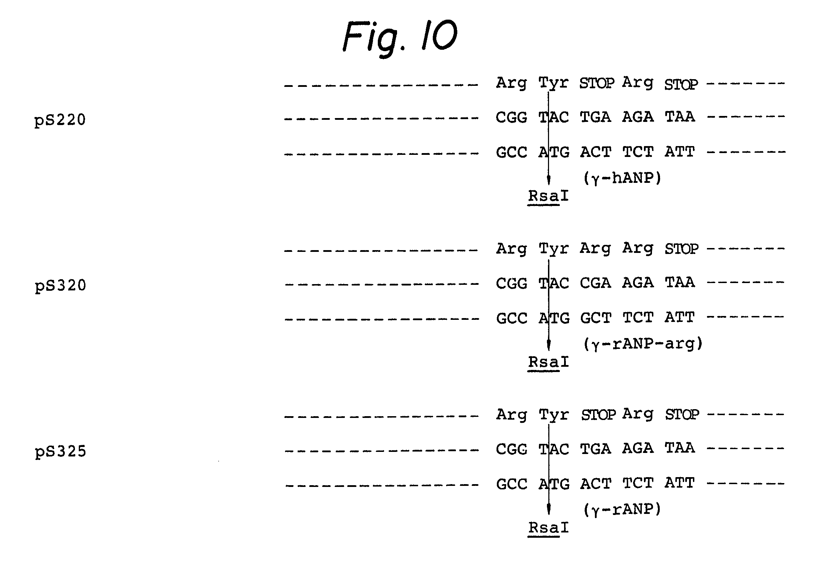

To construct an expression plasmid for γ-rANP, a codon for arginine adjacent to tyrosine, which is the C-terminal amino acid of γ-rANP, has to be converted to a translation stop codon. This conversion was accomplished by deletion of the Aat II - Rsa I fragment containing the γ-hANP gene from the above-mentioned expression plasmid pS220 and by insertion of the Aat II - Rsa I fragment from the above-mentioned expression plasmid pS320 into the plasmid pS220 in place of the deleted Aat II - Rsa I fragment containing the γ-hANP gene to construct an expression plasmid pS325 (Fig. 9). Each region including a codon for C-terminal amino acid tyrosine of γ-hANP or γ-rANP and stop codons in the plasmid pS220, pS320 and pS325 was set forth in Fig. 10. As seen from Fig. 10, by cleavage of pS320 with Rsa I, two codons for arginine and a subsequent base sequence are deleted. By insertion of the Aat II - Rsa I fragment

containing γ-rANP gene into the cleaned plasmid pS220, a codon for tyrosine and an adjacent stop codon are reconstructed. Similarly, an expression plasmid pS329-3 was constructed from the above-mentioned plasmids pS224-3 and pS324-3 (Fig. 9).

Each of the plasmid pS325 and pS329-3 was transformed into E. coli. N4830 to obtain transformant N4830/pS325 and N4830/pS329-3, respectively. The transformants were tested for production of protein according to the procedure as described above for the transformant N4830/pS320 and confirmed to produce a protein having about 20Kd of molecular weight on the SDS-PAGE.

Thus, it was confirmed that γ-rANP, which is a precursor of α-rANP and β-rANP, can be produced by a microorganism using a recombinant gene according to the present invention.

Wherein, although construction of plasmids containing a base sequence coding for an amino acid sequence from 25 to 150 in Fig. 7 (γ-rANP) as a coding region are represented, other plasmids containing a base sequence coding for an amino acid sequence from 1 to 150 in Fig. 7 (signal sequence and γ-rANP) and plasmids containing a base sequence coding for an amino acid sequence from 123 to 150 (α-rANP), an amino acid sequence from 103 to 150 (β-rANP), or any other amino acid sequence as a coding region can be constructed by the same procedure. Moreover, although cDNA corresponding to mRNA prepared from rat atrium cordis was represented as a γ-rANP gene, gene coding for γ-rANP can be chemically synthesized. Moreover, although the λP$_L$ promotor was represented as a promotor region in expression plasmids, other promotors can be used. For example, to express the gene in E. coli, a promotor region of tryptophan gene (trp), lactose gene (lac), lipoprotein gene (lpp), alkaline phosphatase genes (PHOA, PHOS, etc.) or outer membrane protein gene (omp) or a hybrid promotor of tryptophan and lactose promotors (tac promotor) can be used. To express the gene in yeast, a promotor region of alcohol dehydrogenase gene (ADH), glyceraldehyde dehydrogenase gen (GAP-DH), phosphoglycerokinase gene (RGK), or repressible acid phosphatase gene can be used. Moreover, to express the gene in animal cells, or a promotor region of SV40, an early or late gene can be used.

3. Preparation of γ-rANP from rat atrium cordis

γ-rANP can be produced by the extraction of the γ-rANP from rat atrium.

In the process, rat atrium is homogenized in an acidic aqueous solution such as a phosphate buffer solution, or an acetic acid solution containing hydrochloric acid. Subsequently, γ-rANP is purified according to a conventional method suitable for the purification of peptide, such as centrifugation, isoelectric point precipitation, solvent extraction, ultrafiltration, gel filtration, adsorption chromatography, or high performance liquid chromatography (HPLC) or a combination of such methods. In the above-mentioned methods, chick rectum relaxation activity is conveniently used to select fractions containing γ-rANP, because γ-rANP has this activity. In the chromatography methods, the γ-rANP containing fractions can be also selected by molecular weight (about 14,000).

4. Structure and Physico-chemical Properties of γ-rANP

1) Structure:

The γ-ANP has the structure set forth in Fig. 1 excluding -X.

2) Molecular weight: about 13,000 as determined by gel-filtration and about 20,000 as determined by SDS-PAGE (13629 as calculated).

3) UV spectrum: Max = 275 mm.

4) Color reactions: Ehrlich's reaction, negative; Sakaguchi's reaction, positive; Pauly's reaction, positive.

0166585

- 14 -

5) Distinction of basic, acidic, or neutral property: basic.

6) Solubility in solvents: soluble in water, methanol, and acetic acid; insoluble in ethyl acetate, butyl acetate, ethyl ether, hexane, petroleum ether, benzene, and chloroform.

7) Amino acid composition by amino acid analysis:

| Amino acid | Mol. ratio | |
|---|---|---|
| | Found | Calculated |
| Asx | 13.06 | 14 |
| Ala | 9.61 | 10 |
| Arg | 9.58 | 10 |
| Ile | 2.10 | 2 |
| Gly | 11.22 | 12 |
| Glx | 11.18 | 12 |
| $(Cys)_2$ | 0.58 | 1 |
| Ser | 12.31 | 15 |
| Tyr | 1.87 | 2 |
| Phe | 3.30 | 3 |
| Met | 2.82 | 3 |
| Leu | 14.37 | 15 |
| Val | 6.00 | 6 |
| Thr | 2.98 | 3 |
| His | 1.27 | 1 |
| Lys | 4.43 | 4 |

8)    Formation of salts:  the γ-rANP is a basic compound as described in item 5 and can form acid addition salts with an inorganic acid such as hydrochloric acid, sulfuric acid, phosphoric acid, or an organic acid such as formic acid, acetic acid, propionic acid, succinic acid, and citric acid.

5.    Physiological properties of γ-rANP

The γ-rANP according to the present invention has notable diuretic and hypotensive or antihypertensive actions.

Test method:

Male rats weighing 300 to 400 grams were anesthetized by intraperitoneal administration of pentobarbital at a dosage of 60 mg/kg and used for tests on the γ-rANP according to the method described in Life Science, Vol. 28, pp 89-94, 1981, A.J. deBold, et al.

To keep the respiratory tract open, a tracheal cannula (PE-240 Clay-Adams) was inserted into the trachea.  An arterial cannula (PE-50) was inserted into a femoral artery for measurement of the blood pressure, and a venous cannula was inserted into a femoral vein for the administrtion of Ringer's solution. 1.2 ml of Ringer's solution was injected for 10 minutes and, subsequently, was constantly infused at a flow rate of 1.2 ml/hour.

A bladder cannula made of silastic tube with an inner diameter of 0.02 inch and an outer diameter of 0.037 inch was inserted into the bladder, and, via the cannula, a urine sample was collected into a test tube.  The collection of urine was carried out for 30 minutes before administration of the test compound and 5, 10, 20, 30, and 60 minutes after the administration.

One nmole or 2 nmole of the test compound γ-rANP was dissolved in 50 μl of sterilized physiological saline with 5 μg of bacitracin, and the solution was injected into the jugular vein.

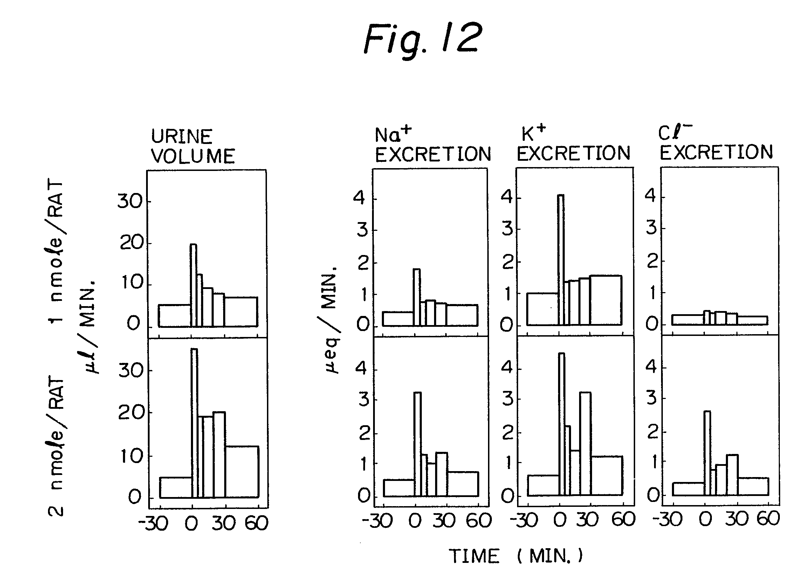

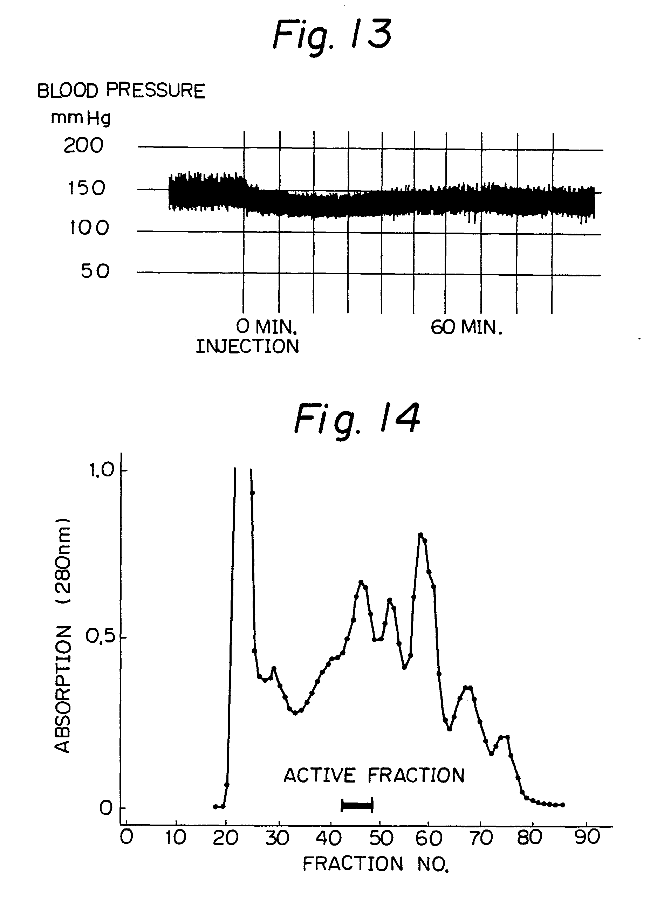

As shown in Fig. 12, γ-rANP shows a notable diuretic action. Moreover, as shown in Fig. 13, after 1 nmole of γ-rANP is administered to a rat, the blood pressure is gradually lowered by about 20 mmHg for about 1 hour, revealing that γ-hANP has a hypotensive action or antihypertensive action, and may be useful as a hypotensor.

6. Use of γ-rANP as a pharmaceutical product

Repeated administration of γ-rANP does not stimulate production of antibodies and does not cause anaphylaxis shock. γ-rANP consisting of L-amino acids is gradually hydrolyzed in a body providing the L-amino acids and therefore shows little toxicity.

Due to the higher diuretic and blook pressure-lowering or antihypertensive actions and the lower toxicity, γ-rANP is useful as an active ingredient for pharmaceutical compositions such as a diuretic and a hypotensor. γ-rANP is administered at 1 μg/kg to 10 mg/kg, preferably 10 μg/kg to 1 mg/kg.

γ-rANP can be administered in the same manner as conventional peptide type pharmaceuticals. Namely, γ-rANP is preferably administered parenterally, for example, intravenously, intramuscularly, intraperito-neally, or subcutaneously. γ-rANP, when administered orally, may be proteolytically hydrolyzed. Therefore, oral application is not usually effective. However, γ-rANP can be administered orally as a formulation wherein γ-rANP is not easily hydrolyzed in a digestive tract, such as liposome-microcapsules. γ-rANP may be also administered in suppositories, sublingual tablets, or intranasal spray.

The parenterally administered pharmaceutical composition is an aqueous solution containig about 0.000005% to 5%, preferably 0.00005% to 0.5%, of γ-rANP, which may contain, in addition to γ-rANP as an active ingredient, for example, buffers such as phosphate and acetate, osmotic pressure-adjusting agents such as sodium chloride, sucrose, and sorbitol, antioxidative or antioxygenic agents, such as ascorbic acid or tochopherol, and preservatives, such as antibiotics. The parenterally administered composition also may be a solution readily usable in a lyophilized form which is dissolved in sterile water before administration.

Examples

The present invention will now be further illustrated by, but is by no means limited to, the following examples.

Example 1.

1)    Preparation of cDNA library, and isolation and identification of α-rANP gene

(1-a)    Preparation of cDNA library

From atrium cordis obtained from 100 Wistar Kyoto rats, 3 mg of RNA was extracted with 4M guanidium thiocyanate according to the method of Chirgwin et al. (Chirgwin, J.M. et. al., Biochemistry 18, 5294-5299, 1979). The RNA was then subjected to an oligo (dT) cellulose column using 10 mM Tris-HCl buffer, pH 7.2, containing 0.5M LiCl, 10 mM EDTA and 0.5% SDS as a binding buffer, and 60 μg of poly (A)$^+$ RNA (mRNA) was isolated (Nakazato, H. & Edmonds, D.S., Meth. Enzym. 29, 431-443, 1974). Twenty μg of the poly (A)$^+$RNA and 4.2 μg of vector primer DNA were used to prepare a cDNA library (plasmids) according to the Okayama-Berg method (Mol. Cell Biol. 2, 161-170, 1983), and the cDNA library was used to transform E. coli WA802. The transformants were screened on an LB-agar medium supplemented with 40 γ/ml of ampicillin. About 60,000 colonies of ampicillin resistant transformants were obtained per microgram of starting mRNA.

(1-b)    Isolation of α-rANP clone

About 15,000 colonies were replicated on a nitrocellulose filter, and the filter was incubated on an LB agar plate supplemented with 40 γ/ml of ampicil-

lin at 37°C for 6 hours. The filter was transferred onto an LB agar plate supplemented with 180 γ/ml of chloramphenicol and incubated at 37°C overnight. The colonies on the filter were lysated with 0.5 N NaOH and neutralized to pH 7.0, and the filter was soaked in 0.5 M Tris -HCl buffer, pH 7.0, containing 1.5 N NaCl, and in 3 x SCC (0.45 M NaCl, 0.45 M sodium citrate) for 5 minutes, respectively. Finally, cell debris on the filter was removed with a paper towel, and the filter was air-dried and then baked at 80°C for 2 hours. The filters were then subjected to hybridization with a probe derived from γ-hANP cDNA clone (plasmid) phANP82. The plasmid phANP82 was disclosed in detail in Japanese Patent Application No. 59-116605. To prepare the probe, the plasmid phANP82 was cleaved with $\underline{Pst}$ I to obtain a $\underline{Pst}$ I - $\underline{Pst}$ I DNA fragment of about 600 bp. The DNA fragment was then nick-translated according to a method described in Rigby, P.W. et. al., J. Mol. Biol., $\underline{113}$, 237, 1977, using α-$^{32}$P dCTP, DNA polymerase I and DNase I, to form the probe, i.e., the DNA fragment labeled with $^{32}$P to a specific activity of 4 x 10$^7$ cpm/pmol. The DNA fragment contains an entire coding region for γ-hANP, whose base sequence is similar to a base sequence of a coding region for γ-rANP. Therefore, the probe is effective for screening γ-rANP genes.

The hybridization was carried out in 3 x SCC containing 1 x Denhardt's solution (0.2% BSA, Armour Pharmaceutical Company; 0.2% Ficol, Sigma; and 0.2% polyvinyl pyrrolidone, Wako Junyaku), 0.1% SDS and 50 μg/ml salmon testis DNA, at 65°C for 16 hours. The filter was then washed with 3 x SCC containing 0.1% SDS at 65°C, air-dried, and placed in contact with an X-ray film. As a result, 50 positive clones were observed on the film. Among the 50 positive clones, 12 clones were used for further investigation. Each plasmid from the 12 clones was cleaved with $\underline{Pvu}$ II and $\underline{Pst}$ I to obtain a DNA fragment which had been originally inserted during the Okayama-Berg method for preparation of the DNA library. The DNA fragments were tested for their molecular weight by gel electrophoresis. Three fragments contained cDNA inserts of 800 bp to 950 pb. The three plasmids containing such cDNA inserts were designated as prANP 10, prANP 11, and prANP 12. The three plasmid DNA's were sequenced using two probes set forth in Fig. 5 as primer according to a dideoxy chain termination method (Sanger F. et al., Proc. Natl. Acad. Sci. USA, 74, 4563-5467, 1977). The primers comprise a mixture of 14-mer oligonucleotides corresponding to an amino acid sequence of from methionine of amino acid position 12 to glycine of amino acid position 16 of α-hANP. As a result, all of the plasmids contained a base sequence corresponding to a part of α-rANP, i.e., an amino acid sequence of from 1 position to 11 position of α-rANP (from amino acid position 123 to amino acid position 133 in Fig. 7). The fact suggests that the above-mentioned three plasmids contain α-rANP genes. Moreover, it was confirmed that the plasmid prANP10 contained the longest cDNA insert.

(1-c)    Identification of γ-rANP gene

The entire base sequence of the cDNA insert was determined according to a strategy shown in Fig. 6. Restriction enzyme sites of the insert cDNA region and subfragments prepared for sequencing are set forth in Fig. 6. In Fig. 6, the direction for sequencing is shown by arrows, wherein solid lines show an upper strand and dotted lines show a lower strand. The sequencing was carried out according to the Maxam-Gilbert method (Meth. Enzym., 65, 499-560, 1980) and Sanger method (Proc. Natl. Acad. Sci, USA, 74, 5463-5467, 1977). A shows the sequencing according to the Maxam-Gilbert method; and B shows the sequencing wherein the plasmid prANP10 was cleaved with PstI and HincII to obtain three fragments. Then, each fragment was inserted into plasmid pUC8 (Vieira, J. & Messing, J., Gene 19, 259-268, 1982) and sequenced according to the Sanger method.

Figure 7 shows the base sequence thus determined of the cDNA insert in the plasmid prANP10 and an amino acid sequence corresponding to an open reading frame in the cDNA sequence. The open reading frame starts from a translation initiation codon ATG coding for methionine at amino acid position 1 and ends at a translation stop codon TAA immediately downstream of a codon for arginine at amino acid position 152. An entire amino acid sequence corresponding to the open reading frame contains an amino acid sequence of α-rANP starting from serine at amino acid position 123 and ending at tyrosine at amino acid position 150; an amino acid sequence of β-rANP starting from prolin at amino acid position 103 and ending at tyrosine at amino acid position 150; and an amino acid sequence of γ-rANP starting from asparagine at amino acid position 25 and ending at tyrosine at amino acid position 150. On the basis of an amino acid sequence of γ-rANP purified from rat atrium cordis and identified, which has an N terminal sequence H-Asn-Pro-Val-Tyr-Ser- ... and C terminal sequence ... -Asn-Ser-Phe-Arg-Tyr-OH as set forth in Fig. 1, the reading frame was confirmed to contain a coding region of from codon AAT of Asn at 25 to codon TAC of Tyr at 150 in Fig. 7.

The reading frame also contains two codons for arginine between the codon for tyrosine at amino acid position 150 and the stop codon TAA. This fact suggests that a precursor of γ-rANP having two additional arginines, designated herein as γ-rANP-arg, is first produced, and the γ-rANP-arg is processed <u>in vivo</u> to form the γ-rANP.

In Fig. 7, an amino acid sequence of from Met at 1 to Ala at 24 is considered from its characteristic sequence to be a signal sequence participating in the secretion of peptide. The base sequence in Fig. 7 also contains a sequence AATAAA shown by solid underlines, which sequence is known to precede the polyadenylation site in many eukaryotic mRNA.

2)    Construction of γ-rANP gene expression vector

(2-a)    Insertion of γ-rANP-arg gene into M13 DNA

0.1 μg of M13mp8 RF-DNA was cleaved with 16 units of PstI in 20 μl of Medium-Salt Buffer (10 mM Tris-HCl buffer, pH 7.5, containing 50 mM NaCl, 10 mM MgCl$_2$ , 1 mM DTT) at 37°C for 2 hours. The mixture was then heated at 65°C for 10 minutes to stop the enzyme reaction. On the other hand, 40 μg of plasmid prANP10 DNA was cleaved with 100 units of PstI in 400 μl of the Medium-Salt Buffer at 37°C for 2 hours, and the reaction mixture was subjected to 1.8% agarose gel electrophoresis. A part of the gel containing a DNA fragment corresponding to about 650 bp was cut to obtain an agarose piece, and the DNA fragment was recovered using DE81 paper and purified.

Twenty ng of the DNA fragment from M13mp8 RF-DNA and 0.2 μg of the 650 bp DNA fragment were ligated using 5.6 units of T4 DNA ligase (Takara Shuzo, Japan) in 20 μl of ligation buffer (20 mM Tris-HCl buffer, pH 7.6, containing 10 mM MgCl$_2$ , 1 mM APT, 5 mM DTT) at 14°C for 16 hours. E. coli JM103 cells were treated with CaCl$_2$ according to a conventinal method to obtain a suspension of the E. coli cells in 50 mM CaCl$_2$ , and the ligation mixture prepared as above (20 μl) was added to 0.3 ml of the E. coli suspension to transform the E. coli.

The transformant clones were screened as follows. The suspension containing transformant E. coli cells was diluted in YT soft agar medium containing X-Gal and IPTG (prepared by adding 10 μl of 10 mM IPTG, 50 μl of 2% X-Gal, and 0.2 ml of E. coli JM103 suspension grown in a logarithmic growth phase into 3 ml of solution containing 0.6% agar, 0.8% bacto trypton, 0.5% yeast extract, and 0.5% NaCl). 3.0 ml of the soft agar medium described above was spread on YT agar medium (1.5% agar, 0.8% Bacto trypton, 0.5% yeast extract, and 0.5% NaCl) and incubated at 37°C for 16 hours to form plaques. Among the plaques, 12 plaques were selected, and inoculated into 2 x YT liquid medium (1.6% Bacto trypton, 1% yeast extract, and 1.0% NaCl) and cultured at 37°C for 8 hours. One ml of the cultured medium was centrifuged at 10,000 rpm for 10 minutes to recover a supernatant containing phage. The phage DNA (single strand DNA) was isolated and purified as follows.

To 800 µl of the phase liquid, 200 µl of 20% polyethylene glycol (PEG 6000) containing 2.5N NaCl was added, and the mixture was allowed to stand at room temperature for 20 minutes and centrifuged at 10,000 rpm for 5 minutes to precipitate the phage. The precipitated phage was dissolved in 100 µl of TE buffer (10 mM Tris-HCl, pH 8.0, 1 mM EDTA). To the solution, 50 µl of phenol saturated with water was added, the mixture was then vigorously stirred for 5 minutes and centrifuged at 10,000 rpm for 5 minutes. After sampling 80 µl of the aqueous phase, to the aqueous phase, 3 µl of 3M sodium acetate, pH 8.0, and 200 µl of ethanol were added. The mixture was cooled at -70°C for 10 minutes and then centrifuged at 10,000 rpm for 10 minutes to precipitate DNA. The precipitated DNA was washed once with ethanol and dissolved in 50 µl of the above-mentioned TE buffer. A part of a base sequence of each phage DNA was sequenced according to the dideoxy chain termination method (Methods in Enzymology, 65, 560-580, 1980, Academic Press, New York). Among 12 clones, 2 clones containing a lower strand (DNA fragment having a base sequence complementary to mRNA) were selected. The phage DNA thus obtained were used as a template for in vitro mutation.

(2-b)    Incorporation of EcoRI cleavage site and translation initiation codon by in-vitro mutation

One µl of the single strand phage DNA solution described above, 1 µl of 0.2M Tris-HCl buffer, pH 7.5, containing 0.1M $MgCl_2$ and 0.5M NaCl, and 4 µl of water, were added to 3 µl of solution containing 0.6 pmole of 33-mer chemically synthesized DNA fragment (5'-CTCCCAGGCCATGAATTCATGAATCCCGTATAC-3') phosphorylated at the 5' end to form 10 µl of a mixture. The mixture was heated at 65°C for 5 minutes and allowed to stand for 10 minutes at room temperature.

To the mixture, 1 µl of 0.2 M Tris-HCl buffer (pH 7.5) containing 0.1 M MgCl, 2 µl of 0.1 M DTT, 1 µl of 10 mM ATP, 2 µl each of 10 mM dATP, dGTP, dCTP, and dTTP, 2 µl of water, 5 units of DNA polymerase I Klenow fragment (Boehringger Manheim) and 2.8 units of T4 DNA ligase (Takara Shuzo) were added, and the mixture was incubated at 15°C for 16 hours. 10 µl of the reaction mixture was used to transform E. coli JM103.

As described above for the preparation of phage DNA, plaques were formed on a YT soft agar medium, and 12 clones were selected. The clones were inoculated to 2 x YT medium and cultured at 37°C for 8 hours. 0.6 ml of the cultured medium was centrifuged at 10,000 rpm for 10 minutes to recover a precipitate. RF-DNA was extracted and isolated from the precipitated cells according to the alkaline extraction method (Birnboim, H.C. & Doly, J., Nucl. Acid. Res., 7, 1513-1523, 1979). The RF-DNA was then cleaved with 4.8 units of EcoRI (Takara Shuzo) in EcoRI buffer (100 mM Tris-HCl, pH 7.5, 50 mM NaCl, 5 mM $MgCl_2$) at 37°C for 2 hours. The reaction mixture was subjected to 2% agarose gel electrophoresis, and a clone providing a DNA fragment of about 520bp was selected. The clone was designated as ml3mp8-rANP10.

To 400 ml of 2 X YT liquid medium, 0.4 ml of culture of E. coli JM103 infected with the above-mentioned phage clone and 4 ml of a not-infected culture of E. coli JM103 were inoculated. The medium was then incubated at 37°C for 8 hours. The cultured medium was centrifuged to obtain an infected cell precipitation and a supernatant phage solution. From the infected cells, RF-DNA was obtained by a density-gradient centrifugation method using cesium chloride and ethidium bromide according to a conventional method. On the other hand, from the supernatant phage solution, phage DNA was obtained, and the phage DNA was sequenced according to the dideoxy chain termination method. As a result, it was confirmed that a translation initiation codon ATG and Eco RI cleavage recognition site GAATTC, i.e., a base sequence GAATTC ATG, were inserted immediately upstream of the γ-rANP-arg gene.

(2-c)    Construction of expression vector for γ-rANP-arg gene (Fig. 8)

An expression vector for a γ-rANP-arg gene was constructed from a plasmid pS220, which is a expression vector for a γ-hANP gene, and a plasmid M13mp-γ-rANP10. A process for construction of pS220 is described in detail thereafter.

Five μg of pS220 DNA was cleaved completely with 15 units of EcoRI and 20 units of SalI, and the reaction mixture was subjected to agar electrophoresis to separate the cleaved fragment. A 4.1 Kd DNA fragment containing a $\lambda P_L$ promotor, a β-lactamase gene, and a replication origin was eluted from the agar by the electro-elution method. The eluted fragment was recovered by ethanol precipitation. On the other hand, 10 μg of RF-DNA of M13mp-γ-rANP10 was cleaved with 30 units of EcoRI and 40 units of SalI, and a DNA fragment of about 500 bp containing γ-rANP-arg gene was obtained according to the above-mentioned method. Both DNA fragments were dissolved together into 20 μl of a ligation buffer (20mM Tris-HCl, pH 7.5, 10mM $MgCl_2$, 1 mM ATP, 10 mM DTT), and the mixture was added with 2 units of T4 DNA ligase and incubated at 15°C for 3 hours. Ten μl of the ligation mixture was added to a suspension of E. coli N4830 in 50 mM $CaCl_2$ , the mixture was allowed to stand at 0°C for 40 minutes to transform the E.coli, and ampicillin resistant clones were obtained according to a conventional procedure. Among the clones, 12 clones were subjected to an alkaline extraction method to test for inserted fragments. All clones were confirmed to contain γ-rANP-arg genes. One of the 12 clones was designated as pS320 and transformed into E. coli N4830 to obtain a transformant E. coli N4830/pS320.

According to a similar procedure, an expression vector pS324 was constructed from a plasmid pS224-3 and the plasmid Ml3mp-γ-rANP10. The starting plasmid pS224-3 is an expression vector for γ-hANP gene and constructed by a process described hereinafter. The plasmid pS324 was transformed into E. coli N4830 to obtain a transformant E. coli N4830/pS324.

The starting plasmid pS220 was constructed from plasmids pS20 and Ml3mp8-hANP525. The plasmid Ml3mp8-hANP525 contains a DNA fragment of about 510 bp flanked by an EcoRI site and SalI site which DNA fragment contains γ-hANP gene (Japanese Patent Application No. 59-116605).

The starting plasmid pS20 was constructed by the inventors, and E. coli N4380/pS20 containing the plasmid pS20 was designated as SBM271 and deposited as FERM BP-535 as described above. The plasmid pS20 contains λ phage $P_L$ promotor and can express a foreign gene inserted to a site downstream of the promotor under the control of the promotor.

The plasmid pS20 was constructed as follows. Bacteriophage λC1857 DNA was cleaved with BamHI and Hind III to obtain a 2.4 kb DNA fragment containing the $\lambda P_L$ promotor. The DNA fragment was inserted into a region of Hind III-Bam HI in pBR322 to obtain a plasmid which is substantially the same as the plasmid pKO 30 described in Nature 292, 128, 1981. To the Hpa I cleavage site of the plasmid thus obtained, a 1.3 Kb Hae III DNA fragment containing NutR, $tR_1$ , CII and a part of O protein derived from bacteriophage λcy3048 (from Dr. Hiroyuki Shimatake, Medical Department, Toho University) was inserted to obtain plasmid (pS9), wherein CII is present in the direction the same as the transcription direction of $\lambda P_L$. The plasmid (pS9) was cleaved with Bgl II and Rsa I to obtain a 0.65 Kb DNA fragment containing PL promotor, DNA sequence of protein N', which is a part of an N protein lacking a C terminal, and the Shine-Dalgarno sequence (SD) of a CII gene. The Rsa I end of the 0.65 Kb DNA fragment was added with the Eco RI linker:

-CGGAATTCCG-

-GCCTTAAGGC-

(New England Biolabos, Inc.), and then the Bgl II end of the same DNA fragment was converted to a blunt end with T4 DNA polymerase. The DNA fragment thus obtained was ligated with a DNA fragment prepared by Eco RI cleavage of plasmid pBR322 and conversion of the pBR322 ends to blunt ends to form a plasmid (pS13). In the plasmid (pS13), the $P_L$ promotor is oriented in the direction the same as the transcription direction of the tetracycline resistant gene ($Tc^r$) derived from pBR322. The plasmid (pS13) was cleaved with Eco RI and Sal I, and a large fragment was isolated. The large fragment was then ligated with a DNA fragment containing a foreign gene, i.e., human γ-interferon gene GIF, which fragment was prepared by cleavage of plasmid pGIF4 with Eco RI and Sal I. The plasmid pGIF4 was disclosed in Japanese Unexamined Patent Publication No. 58-201995 (USP. Serial No. 496176), and E. Coli containing the plasmid was designated as SBMG 105 and deposited at the FRI under the Budapest Treaty on the International Recognition of the Deposit of Microorganisms for the Purpose of Patent Procedure as FERM BP-282 on May 6, 1982.

Starting with the plasmid pS20 and

M13mp8-hANP525, the γ-hANP expression vector pS220 was constructed as follows. To 70 µl of Eco RI reaction buffer (100 mM Tris-HCl, pH 7.3, 50 mM NaCl, 5 mM MgCl$_2$) containing 8 µg of M13mp8-hANP525 DNA, 15 units of Eco RI and 15 units of Sal I were added, the reaction mixture was incubated at 37°C for 1 hour, and then subjected to the 1.0% agar gel electrophoresis. About 510 bp Eco RI - Sal I DNA fraction containing γ-hANP gene was extracted from the agar gel and purified by the electroelution method described above. On the other hand, to 50 µl of Eco RI reaction buffer containing 2 µg of plasmid pS20 DNA, 10 units of Eco RI and 10 units of Sal I were added, and the reaction mixture was incubated at 37°C for 1 hour, and then subjected to 1.0% agar gel electrophoresis. According to the above-mentioned electroelution, about 4.3 Kb Eco RI - Sal I DNA fragment containing the λP$_L$ promotor and the SD sequence of the CII gene was obtained. Three µl of an aqueous solution of the Eco RI - Sal I DNA fragment containing γ-hANP gene and 10 µl of an aqueous solution of the Eco RI - Sal I DNA fragment derived from plasmid pS20 were mixed. To the mixture, 3 µl of ligation buffer with 10 times the concentration, 3 µl of 100 mM DTT, 3 µl of 1 mM ATP aqueous solution, 6 µl of distillated water, and 2 units of T4 DNA ligase were added, and the mixture was incubated at 16°C overnight to obtain a ligation mixture. The ligation mixture was used to transform E. coli N4830 (from Pharmacia P-L Biochemicals) as follows. The E. coli cells were treated according to a conventional procedure to obtain competent cells. To 300 µl of the treated E. coli cell suspension containing 50 mM CaCl$_2$ , 10 µl of the ligation mixture obtained as above was added, and the resulting mixture was allowed to stand for 1 hour in ice. 2.5 ml of L-broth was added to the mixture, which was then incubated at 32°C for 1.5 hours. The resulting mixture was plated on a nutrient agar medium containing 50 µg/ml of ampicillion to select ampicillin resistant colonies which are transformants. The transformants were treated according to a conventional procedure to isolate plasmid DNA's. The plasmids were screened by analysis using restriction enzymes. A plasmid having the γ-hANP gene downstream of the λP$_L$ promotor was selected and designated as pS220. The transformant containing the plasmid pS220 was designated as E. coli N4380/pS220 and was used in the test for γ-hANP production as described below in detail as positive control.

In addition to the starting plasmid pS220, other starting plasmids were constructed as follows. Plasmid pS20 was cleaved with Eco RI and hydrolyzed with an exonuclease Bal 31 to form DNA fragments having various lengths. The DNA fragments were ligated with Xba I linker (from New England Biolabos Inc.; dCTCTAGAG) to obtain plasmid pS20X. The plasmid pS20X was cleaved with Xba I and Sal I to delete any Xba I - Sal I short fragments. On the other hand, plasmid pIN4GIF54 was cleaved with Xba I and SalI to obtain a short Xba I - Sal I fragment containing human γ-interferon gene, which short fragment was then inserted to the above-mentioned cleaved pS20X in place of the deleted Xba I - Sal I fragment to form plasmids. Among the plasmids thus formed, a plasmid which can effectively express the human γ-interferon gene when transformed into E. coli was designated as pS83-3. The above-mentioned plasmid pIN4GIF54 and a method for measuring an amount of γ-interferon were disclosed in Japanese Unexamined Patent Publication No. 60-24187. The plasmid pS83-3 was cleaved with Xba I and Eco RI to delete the SD sequence of the lpp gene. In place of the delected SD sequence of the lpp gene, a chemically synthesized DNA fragment AGGAGGT with Xba I and Eco RI cohesive ends, which is the SD sequence of the bacteriophage MS2 A protein gene, was inserted into the cleaved pS83-3 to form plasmid pS84-3. The plasmid pS84-3 DNA was cleaved with Eco RI and Sal I to delete the Eco RI - Sal I short fragment consisting of GIF. The plasmid M13mp8-hANP525 DNA was cleaved with Eco RI and Sal I to obtain an about 510 bp Eco RI - Sal I fragment containing the γ-hANP gene, which fragment was then inserted into the cleaved pS84-3 DNA in place of the deleted Eco RI - Sal I fragment consisting of GIF to obtain plasmid pS224-3. The plasmid pS224-3 thus obtained contained a λP$_L$ promotor region, SD sequence of MS2A, and γ-hANP gene, in this order. E. coli N4380 transformed with the plasmid pS224-3 was designated as E. coli N4380/pS224-3.

(2-d)    Construction of expression vector for γ-rANP gene (Fig. 9)

Five μg of the plasmid pS220 DNA was cleaved with 15 units of Aat II and 15 units of Ava I to obtain a DNA fragment of about 2.9 Kb containing a β-lactomase gene and a replication origin according to the above-mentioned method. In addition, 5 μg of pS220 DNA was cleaved with 15 units of Ava I and 15 units of Rsa I to obtain a DNA fragment of about 0.9 Kb containing translation stop codons of γ-hANP gene. On the other hand, 5 μg of pS320 DNA was cleaved with 15 units of Aat II and 15 unis of Rsa I to obtain a DNA fragment of about 0.7 Kb containing γ-rANP. The three DNA fragments thus obtained were mixed and dissolved in 20 μl of the ligation buffer. The mixture was added with 10 units of T4 DNA ligase and incubated at 15°C for 16 hours. Ten μl of the ligation mixture was added to 0.3 ml of a suspension of E. coli N4830 in 50 mM CaCl$_2$ to transform the E. coli. The transformants thus obtained were screened for ampicillin resistance, and the amicillin resistant transformants thus selected were tested for their correct sequence. One of the transformants was selected and designated as E. coli N4830/pS325. The transformant contains an expression plasmid for γ-rANP gene designated as pS325.

According to a similar procedure, another expression vector for γ-rANP gene, i.e., pS329-3, was constructed from plasmids pS224-3 and pS324-3 and transformed into E. coli N4830 to obtain a transformant E. coli N4830/pS329-3.

3) Production of γ-rANP and γ-rANP-arg by transformant

Transformants E. coli N4830/pS320 and N4830/pS324-3 for production of γ-rANP-arg; E. coli N4830/pS325 and N4830/pS329-3 for production of γ-rANP; and a positive control transformant N4830/pS220, and a negative control strain E. coli N4830 containing no plasmid were compared. Each of these six strains was cultured in an M9 minimum medium supplemented with 50 µg/ml of amplicillin and 19 natural amino acids except for L-methionine at 32°C until the optical density at 660 nm (OD 660) of the culture reached 0.4. At this point, each culture was divided into two cultures. One of the divided cultures was cultured at the same temperature, i.e., 32°C; and the other was cultured at 42°C. At 15 minutes and 30 minutes after the division of the culture, 0.3 ml of culture was obtained from each culture, and to the culture (0.3 ml), 2 µCi of $^{35}$S-methionine with 800 Ci/m mole of specific activity (New England Nuclea) was added. Culturing was continued for 2 minutes and then trichloroacetic acid was added to the contentration of 10% to each culture, which was then cooled to 0°C. The mixture was then centrifuged for 10 minutes, and the precipitate was washed once with ethanol and subjected to 15% SDS polyacrylamide gel electrophoresis to separate proteins. The gel was dried and subjected to a radioautography to detect bands of proteins labeled with $^{35}$S-methionine. Part of the results is shown in Fig. 10.

As a result, strains N4830/pS320 and N4830/pS324-3 incubated at 42°C produced a protein which provides a band corresponding to a molecular weight slightly higher than 20 Kb on radioautography. Strains N4830/pS325, N4830/pS329-3, and N4830/pS220 incubated at 42°C produced a protein which provides a band corresponding to a molecular weight of about 20 Kb on radioautography. On the other hand, all strains incubated at 32°C after division of culture and the negative control strain produced no proteins providing a band corresponding to a molecular weight of about 20 Kd or thereupon on radioautography. As the $\lambda P_L$ promotor is activated at an elevated temperatue, the above-mentioned fact means that a gene inserted into the downstream site of a $\lambda P_L$ promotor was expressed at 42°C under the control of the $\lambda P_L$ promotor.

The molecular weight of about 20 Kb is higher than the calculated molecular weight of γ-rANP or γ-rANP-arg. On the basis of the fact that γ-rANP prepared from rat atrium cordis migrates to a site the same as the expression product of N4830/pS325 and N4830/329-3 on SDS polyacrylamide gel electrophoresis under the same condition and the fact an SDS polyacrylamide gel electrophoresis often shows a molecular weight higher than the actual molecular weight, it is strongly suggested that the E. coli N4830/pS320 and N4830/pS324-3 produced γ-rANP-arg, and N4830/pS325 and N4830/pS329-3 produced γ-rANP.

In the SDS polyacrylamide gel electrophoresis, a standard protein kit for determination of the moleculor weight supplied by Pharmacia P-L Biochemicals was used.

Example 2. Preparation of γ-rANP from rat atrium cordis

16.9 g of rat atrium cordis was obtained and added with ten volumes of 1 M acetic acid aqueous solution. The mixture was homogenized and diluted to two volumes with cold acetone. The homogenate thus obtained was centrifuged at 16000 XG for 30 minutes to obtain a supernatant. From the supernatant, acetone was removed by vacuum distillation to obtain 100 ml of an extract containing γ-rANP.

To the extract, 450 ml of 1 N acetic acid solution was added, and the diluted extract was subjectd to reverse column chromatography using 90 ml of LCSORB SP-C-ODS column (Chemco). The charged column was washed with 400 ml of 0.1 N acetic acid solution and eluted with 300 ml of a mixed eluent of water: acetonitrile: 10% trifluoroacetic acid (40:60:1). The elute was lyophylized to obtain 120 mg of dried residue.

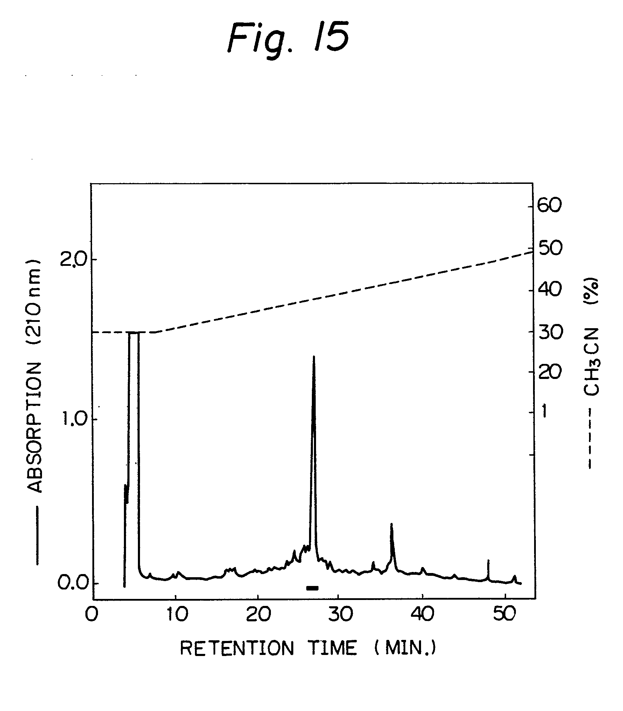

The residue thus obtained was dissolved in 5 ml of 1 N acetic acid, and the solution was subjected to gel filtration using a Sephadex G-75 column (Pharmacia, 1.8 x 135 cm) equilibrated with a 1 N acetic acid solution. The elution was carried out with 1 N acetic acid, and 5 ml of fractions were obtained. The elution profile is set forth in Fig. 14. Fractions No. 43 to 48 having chicken rectum relaxation activity were combined, and 100 μl of the combined fraction was subjected to HPLC using a TSK ODS column (Toyo Soda, 4.0 x 250 mm) and two eluents, i.e., (A) a mixture of water: acetonitrile: 10% trifluoroacetic acid (90:10:1) and (B) a mixture of water: acetonitrile: 10% acetic acid (40:60:1). The column was equilibrated with eluent (A), and 250 μl of the active fraction obtained from the Sephadex G-75 column was applied to the column. Elution was carried out by linear gradient from (A) to (B) at a flow rate of 1.0 ml/min and at a pressure of 110 to 130 $kg/cm^2$ for 80 minutes. The result is set forth in Fig. 15. A peak fraction having chicken rectum relaxation activity (retention time 50 minutes) was obtained and subjected to HPLC using the same column. The same procedure was repeated to obtain 266 nmole of γ-rANP.

Example 3. <u>Preparation of parenteral composition</u>

A) Injection solution

<u>Composition</u>

| | |
|---|---|
| γ-rANP | 2 g |
| sodium chloride | 8 g |
| ascorbic acid | 2 g |

sterile water        1

Method

γ-rANP and sodium chloride were dissolved in sterile water, an ampule was filled with 5 ml of the solution, and the ampule was then sealed.

B)    Lyophilizate

Composition

γ-rANP          2 g

sorbital       20 g

Method

γ-rANP and sorbital were dissolved in 200 ml of sterile water, a vial was filled with 1 ml of the solution and lyophilized, and the vial was then sealed.

The composition is dissolved in 5 ml of sterile water before parenteral administration.

CLAIMS

1. A DNA fragment comprising a base sequence coding for a peptide occurring in rat atrium cordis and having diuretic action or a precursor of the peptide.

2. A DNA fragment according to claim 1, wherein the base sequence codes for a peptide consisting of all or a part of the following amino acid sequence:

Met Gly Ser Phe Ser Ile Thr Lys Gly Phe
Phe Leu Phe Leu Ala Phe Trp Leu Pro Gly
His Ile Gly Ala Asn Pro Val Tyr Ser Ala
Val Ser Asn Thr Asp Leu Met Asp Phe Lys
Asn Leu Leu Asp His Leu Glu Glu Lys Met
Pro Val Glu Asp Glu Val Met Pro Pro Gln
Ala Leu Ser Glu Gln Thr Asp Glu Ala Gly
Ala Ala Leu Ser Ser Leu Ser Glu Val Pro
Pro Trp Thr Gly Glu Val Asn Pro Ser Gln
Arg Asp Gly Gly Ala Leu Gly Arg Gly Pro
Trp Asp Pro Ser Asp Arg Ser Ala Leu Leu
Lys Ser Lys Leu Arg Ala Leu Leu Ala Gly
Pro Arg Ser Leu Arg Arg Ser Ser Cys Phe
Gly Gly Arg Ile Asp Arg Ile Gly Ala Gln
Ser Gly Leu Gly Cys Asn Ser Phe Arg Tyr X

wherein X is absent or represents up to two arginine residues.

3. A DNA fragment according to claim 2, wherein the peptide has the following amino acid sequence:

Asn Pro Val Tyr Ser Ala Val Ser Asn Thr
Asp Leu Met Asp Phe Lys Asn Leu Leu Asp
His Leu Glu Glu Lys Met Pro Val Glu Asp
Glu Val Met Pro Pro Gln Ala Leu Ser Glu
Gln Thr Asp Glu Ala Gly Ala Ala Leu Ser
Ser Leu Ser Glu Val Pro Pro Trp Thr Gly
Glu Val Asn Pro Ser Gln Arg Asp Gly Gly
Ala Leu Gly Arg Gly Pro Trp Asp Pro Ser
Asp Art Ser Ala Leu Leu Lys Ser Lys Leu
Arg Ala Leu Leu Ala Gly Pro Arg Ser Leu
Arg Arg Ser Ser Cys Phe Gly Gly Arg Ile

Asp Arg Ile Gly Ala Gln Ser Gly Leu Gly
Cys Asn Ser Phe Arg Tyr X

wherein X is absent or represents up to two arginine residues.

4.    A DNA fragment according to claim 2, wherein the base sequence consists of all or a part of the following sequence:

```
ATG GGC TTC TTC TCC ATC ACC AAG GGC TTC
TTC CTC TTC CTG GCC TTT TGG CTC CCA GGC
CAT ATT GGA GCA AAT CCC GTA TAC AGT GCG
GTG TCC AAC ACA GAT CTG ATG GAT TTC AAG
AAC CTG CTA GAC CAC CTG GAG GAG AAG ATG
CCG GTA GAA CAT GAG GTC ATG CCT CCG CAG
GCC CTG AGC GAG CAG ACC GAT GAA GCG GGG
GCG GCA CTT AGC TCC CTC TCT GAG GTG CCT
CCC TGG ACT GGG GAA GTC AAC CCG TCT CAG
AGA GAT GGA GGT GCT CTC GGG CGC GGC CCC
TGG GAC CCC TCC GAT AGA TCT GCC CTC TTG
AAA AGC AAA CTG AGG GCT CTG CTC GCT GGC
CCT CGG AGC CTG CGA AGG TCA AGC TGC TTC
GGG GGT AGG ATT GAC AGG ATT GGA GCC CAG
AGC GGA CTA GGC TGC AAC AGC TTC CGG TAC Y
```

wherein Y is absent or represents up to two codons coding for arginine.

5.    A DNA fragment according to claim 3, wherein the peptide is coded by the following sequence:

```
AAT CCC GTA TAC AGT GCG GTG TCC AAC ACA
GAT CTG ATG GAT TTC AAG AAC CTG CTA GAC
CAC CTG GAG GAG AAG ATG CCG GTA GAA GAT
GAG GTC ATG CCT CCG CAG GCC CTG AGC GAG
CAG ACC GAT GAA GCG GGG GCG GCA CTT AGC
TCC CTC TCT GAG GTG CCT CCC TGG ACT GGG
GAA GTC AAC CCG TCT CAG AGA GAT GGA GGT
GCT CTC GGG CGC GGC CCC TGG GAC CCC TCC
GAT AGA TCT GCC CTC TTG AAA AGC AAA CTG
AGG GCT CTG CTC GCT GGC CCT CGG AGC CTG
CGA AGG TCA AGC TGC TTC GGG GGT AGG ATT
```

```
GAC AGG ATT GGA GCC CAG AGC GGA CTA GGC
TGC AAC AGC TTC CGG TAC Y
```

wherein Y is absent or represents up to two codons
coding for arginine.

6.    A DNA fragment according to any one of the
preceding claims wherein the DNA fragment is cDNA
prepared by using mRNA as a template, which mRNA is
obtained from rat atrium cordis.

7.    A plasmid containing a promotor regin, SD
sequence, and a DNA fragment defined in any one of
the preceding claims.

8.    A plasmid according to claim 7, wherein
the promotor is a $\lambda P_L$ promotor.

9.    A plasmid according to claim 7 or claim
8 wherein the SD sequence is an SD sequence of a CII
gene, SD sequence of an lpp gene, or SD sequence of
an MS2A gene.

10.    A plasmid according to claim 7, wherein
the plasmid is pS320, pS324-3, pS325, or pS329-3.

11.    A microorganism transformed with a plasmid
defined in any one of claims 7 to 10.

12.    A microorganism according to claim 11, wherein
the microorganism is E. coli.

13.    A process for production of a peptide occurring
in rat atrium cordis and having diuretic action and/or
a   precursor of the peptide, comprising culturing
a microorganism defined in claim 11 to form the peptide
or precursor peptide and recovering the respective
peptide;

converting such a peptide or precursor to an
acid addition salt thereof; or

converting an acid addition salt of such a peptide
or precursor to the free base form thereof.

14.    A precursor of a diuretic peptide γ-rANP of
the following amino acid sequence:

Met Gly Ser Phe Ser Ile Thr Lys Gly Phe
Phe Leu Phe Leu Ala Phe Trp Leu Pro Gly
His Ile Gly Ala Asn Pro Val Tyr Ser Ala
Val ser Asn Thr Asp Leu Met Asp Phe Lys
Asn Leu Leu Asp His Leu Glu Glu Lys Met
Pro Val Glu Asp Glu Val Met Pro Pro Gln
Ala Leu Ser Glu Gln Thr Asp Glu Ala Gly
Ala Ala Leu Ser Ser Leu Ser Glu Val Pro
Pro Trp Thr Gly Glu Val Asn Pro Ser Gln
Arg Asp Gly Gly Ala Leu Gly Arg Gly Pro
Trp Asp Pro Ser Asp Arg Ser Ala Leu Leu
Lys Ser Lys Leu Arg Ala Leu Leu Ala Gly
Pro Arg Ser Leu Arg Arg Ser Ser Cys Phe
Gly Gly Arg Ile Asp Arg Ile Gly Ala Gln
Ser Gly Leu Gly Cys Asn Ser Phe Arg Tyr X

wherein X is absent or represents up to two arginine residues, and an acid addition salt thereof.

15. A diuretic peptide γ-rANP of the following amino acid sequence:

Asn Pro Val Tyr Ser Ala Val Ser Asn Thr
Asp Leu Met Asp Phe Lys Asn Leu Leu Asp
His Leu Glu Glu Lys Met Pro Val Glu Asp
Glu Val Met Pro Pro Gln Ala Leu Ser Glu
Gln Thr Asp Glu Ala Gly Ala Ala Leu Ser
Ser Leu Ser Glu Val Pro Pro Trp Thr Gly
Glu Val Asn Pro Ser Gln Arg Asp Gly Gly
Ala Leu Gly Arg Gly Pro Trp Asp Pro Ser
Asp Arg Ser Ala Leu Leu Lys Ser Lys Leu
Arg Ala Leu Leu Ala Gly Pro Arg Ser Leu
Arg Arg Ser Ser Cys Phe Gly Gly Arg Ile
Asp Arg Ile Gly Ala Gln Ser Gly Leu Gly
Cys Asn Ser Phe Arg Tyr

and an acid addition salt thereof.

16. A diuretic composition containing a peptide γ-rANP defined in claim 15 or an acid addition salt thereof with a conventional pharmaceutical additive.

0166585

- 38 -

17.   A hypotensor composition containing a peptide $\gamma$-rANP defined in claim 15, or an acid addition salt thereof, with a conventional pharmaceutical additive.

18.   A composition according to claim 16 or claim 17, wherein the composition is a solution for parenteral administration and the additive is a buffer, an osmotic pressure adjusting agent, or a preservative or a combination thereof.

19.   A composition according to any one of claims 16 to 18 wherein the composition is a solution for parenteral administration and contains about 0.000005% to 5% of the $\gamma$-rANP.

20.   A composition according to any one of claims 16 to 19 wherein the composition is in a lyophylized form.

21.   The medical use of $\gamma$-rANP as defined in claim 15, or an acid addition salt thereof, or a composition according to any one of claims 16 to 20.

## Fig. 1

```
1                                          10                                        20
Asn Pro Val Tyr Ser Ala Val Ser Asn Thr Asp Leu Met Asp Phe Lys Asn Leu Leu Asp

                                           30                                        40
His Leu Glu Glu Lys Met Pro Val Glu Asp Glu Val Met Pro Pro Gln Ala Leu Ser Glu

                                           50                                        60
Gln Thr Asp Glu Ala Gly Ala Ala Leu Ser Ser Leu Ser Glu Val Pro Pro Trp Thr Gly

                                           70                                        80
Glu Val Asn Pro Ser Gln Arg Asp Gly Gly Ala Leu Gly Arg Gly Pro Trp Asp Pro Ser

                                           90                                        100
Asp Arg Ser Ala Leu Leu Lys Ser Lys Leu Arg Ala Leu Leu Ala Gly Pro Arg Ser Leu

                                           110                                       120
Arg Arg Ser Ser Cys Phe Gly Gly Arg Ile Asp Arg Ile Gly Ala Gln Ser Gly Leu Gly

                         126
Cys Asn Ser Phe Arg Tyr  -X
```

## Fig. 2

Met Gly Ser Phe Ser Ile Thr Lys Gly Phe Phe Leu Phe Leu Ala Phe Trp Leu Pro Gly

His Ile Gly Ala Asn Pro Val Tyr Ser Ala Val Ser Asn Thr Asp Leu Met Asp Phe Lys

Asn Leu Leu Asp His Leu Glu Glu Lys Met Pro Val Glu Asp Glu Val Met Pro Pro Gln

Ala Leu Ser Glu Gln Thr Asp Glu Ala Gly Ala Ala Leu Ser Ser Leu Ser Glu Val Pro

Pro Trp Thr Gly Glu Val Asn Pro Ser Gln Arg Asp Gly Gly Ala Leu Gly Arg Gly Pro

Trp Asp Pro Ser Asp Arg Ser Ala Leu Leu Lys Ser Lys Leu Arg Ala Leu Leu Ala Gly

Pro Arg Ser Leu Arg Arg Ser Ser Cys Phe Gly Gly Arg Ile Asp Arg Ile Gly Ala Gln

Ser Gly Leu Gly Cys Asn Ser Phe Arg Tyr　—X

## Fig. 3

AAT CCC GTA TAC AGT GCG GTG TCC AAC ACA GAT CTG ATG GAT TTC AAG AAC CTG CTA GAC    60

CAC CTG GAG GAG AAG ATG CCG GTA GAA GAT GAG GTC ATG CCT CCG CAG GCC CTG AGC GAG   120

CAG ACC GAT GAA GCG GGG GCG GCA CTT AGC TCC CTC TCT GAG GTG CCT CCC TGG ACT GGG   180

GAA GTC AAC CCG TCT CAG AGA GAT GGA GGT GCT CTC GGG CGC GGC CCC TGG GAC CCC TCC   240

GAT AGA TCT GCC CTC TTG AAA AGC AAA CTG AGG GCT CTG CTC GCT GGC CCT CGG AGC CTG   300

CGA AGG TCA AGC TGC TTC GGG GGT AGG ATT GAC AGG ATT GGA GCC CAG AGC GGA CTA GGC   360

TGC AAC AGC TTC CGG TAC   -Y

## Fig. 4

```
ATG GGC TCC TTC TCC ATC ACC AAG GGC TTC TTC CTC TTC CTG GCC TTT TGG CTC CCA GGC    60

CAT ATT GGA GCA AAT CCC GTA TAC AGT GCG GTG TCC AAC ACA GAT GTG ATG GAT TTC AAG   120

AAC CTG CTA GAC CAC CTG GAG GAG AAG ATG CCG GTA GAA GAT GAG GTC ATG CCT CCG CAG   180

GCC CTG AGC GAG CAG ACC GAT GAA GCG GGG GCG GCA CTT AGC TCC CTC TCT GAG GTG CCT   240

CCC TGG ACT GGG GAA GTC AAC CCG TCT CAG AGA GAT GGA GGT GCT CTC GCG CGC GGC CCC   300

TGG GAC CCC TCC GAT AGA TCT GCC CTC TTG AAA AGC AAA CTG AGG GCT CTG CTC GCT GGC   360

CCT CGG AGC CTG CGA AGG TCA AGC TGC TTC GGG GGT AGG ATT GAC AGG ATT GGA GCC CAG   420

AGC GGA CTA GGC TGC AAC AGC TTC CGG TAC   -Y
```

## Fig. 5

Met Asp Arg Ile Gly

3'- TAC CT$_A^G$ GC$_A^{T\ G\ C}$ TA$_A^{T}$G CC-5'     Probe I

3'- TAC CT$_A^G$ TC$_C^T$ TA$_A^T$G CC-5'     Probe II

## Fig. 8

Fig. 6

Bgl II (103)

Ban I (235)
Hinc II (259)
Ava II (304)
Bgl II (317)

Ban I (447)
Rsa I (449)

Pst I (568)

Aat I (626)

-100  0  100  200  300  400  500  600  700  800

A

B

# Fig. 7A

```
                              -57
              5'-CAACCAGAGAGTGAGCCGAGACAGCAAACATCAGATCGTGCCCCGACCCACGCCAGC    -1

                                       10                                          20
Met Gly Ser Phe Ser Ile Thr Lys Gly Phe Phe Leu Phe Leu Ala Phe Trp Leu Pro Gly
ATG GGC TCC TTC TCC ATC ACC AAG GGC TTC TTC CTC TTC CTG GCC TTT TGG CTC CCA GGC    60

                                       30                                          40
His Ile Gly Ala Asn Pro Val Tyr Ser Ala Val Ser Asn Thr Asp Leu Met Asp Phe Lys
CAT ATT GGA GCA AAT CCC GTA TAC AGT GCG GTG TCC AAC ACA GAT CTG ATG GAT TTC AAG   120

                                       50                                          60
Asn Leu Leu Asp His Leu Glu Glu Lys Met Pro Val Glu Asp Glu Val Met Pro Pro Gln
AAC CTG CTA GAC CAC CTG GAG GAG AAG ATG CCG GTA GAA GAT GAG GTC ATG CCT CCG CAG   180

                                       70                                          80
Ala Leu Ser Glu Gln Thr Asp Glu Ala Gly Ala Ala Leu Ser Ser Leu Ser Glu Val Pro
GCC CTG AGC GAG CAG ACC GAT GAA GCG GGG GCG GCA CTT AGC TCC CTC TCT GAG GTG CCT   240

                                       90                                         100
Pro Trp Thr Gly Glu Val Asn Pro Ser Gln Arg Asp Gly Gly Ala Leu Gly Arg Gly Pro
CCC TGG ACT GGG GAA GTC AAC CCG TCT CAG AGA GAT GGA GGT GCT CTC GGG CGC GGC CCC   300
```

## Fig. 7B

```
                                       110                                          120
Trp Asp Pro Ser Asp Arg Ser Ala Leu Leu Lys Ser Lys Leu Arg Ala Leu Leu Ala Gly
TGG GAC CCC TCC GAT AGA TCT GCC CTC TTG AAA AGC AAA CTG AGG GCT CTG CTC GCT GGC   360

                                       130                                          140
Pro Arg Ser Leu Arg Arg Ser Ser Cys Phe Gly Gly Arg Ile Asp Arg Ile Gly Ala Gln
CCT CGG AGC CTG CGA AGG TCA AGC TGC TTC GGG GGT AGG ATT GAC AGG ATT GGA GCC CAG   420

                                       150        152
Ser Gly Leu Gly Cys Asn Ser Phe Arg Tyr Arg Arg
AGC GGA CTA GGC TGC AAC AGC TTC CGG TAC CGA AGA TAA CAGCCAAATCTGCTCGAGCAGATCGCA   486

AAAGATCCCAAGCCTTGCGGTGTGTCACACAGCTTGGTCGCATTGCCACTGAGAGGTGGTGAATACCCTCCTGGAGCTG   565

CAGCTTCCTGTCTTCATCTATCACGATCGATGTTAAGTGTAGATGAGTGGTTTAGTGAGGCCTTACCTCTCCCACTCTG   644

CATATTAAGGTAGATCCTCACCCCTTTCAGAAAGCAGTTTGGAAAAAAATAAATCCGAATAAACTTCAGCACCACGGAC   723

AG-3'
```

*Fig. 9*

# Fig. 10

pS220

```
-----------------  Arg Tyr STOP Arg STOP --------
-----------------  CGG TAC TGA AGA TAA --------
-----------------  GCC ATG ACT TCT ATT --------
                             (γ-hANP)
                   RsaI
```

pS320

```
-----------------  Arg Tyr Arg Arg STOP --------
-----------------  CGG TAC CGA AGA TAA --------
-----------------  GCC ATG GCT TCT ATT --------
                             (γ-rANP-arg)
                   RsaI
```

pS325

```
-----------------  Arg Tyr STOP Arg STOP --------
-----------------  CGG TAC TGA AGA TAA --------
-----------------  GCC ATG ACT TCT ATT --------
                             (γ-rANP)
                   RsaI
```

Fig. 11

Fig. 12

# Fig. 13

BLOOD PRESSURE
mmHg

200

150

100

50

O MIN.
INJECTION

60 MIN.

# Fig. 14

1.0

ABSORPTION (280nm)

0.5

0

ACTIVE FRACTION

0   10   20   30   40   50   60   70   80   90

FRACTION NO.

# Fig. 15